(19) Europäisches Patentamt
European Patent Office
Office européen des brevets

(11) Publication number: **0 104 197**
**B1**

(12) **EUROPEAN PATENT SPECIFICATION**

(45) Date of publication of patent specification: **28.05.86**

(51) Int. Cl.⁴: **C 07 C 29/136**, C 07 C 51/12, C 07 C 67/08, C 07 C 31/08, C 07 C 53/08, C 07 C 69/14

(21) Application number: **83900951.1**

(22) Date of filing: **22.03.83**

(86) International application number: **PCT/GB83/00085**

(87) International publication number: **WO 83/03409 13.10.83 Gazette 83/24**

(54) **PROCESS FOR THE PRODUCTION OF ETHANOL.**

(30) Priority: **26.03.82 GB 8208943**

(43) Date of publication of application: **04.04.84 Bulletin 84/14**

(45) Publication of the grant of the patent: **28.05.86 Bulletin 86/22**

(84) Designated Contracting States: **AT BE CH DE FR GB LI LU NL SE**

(56) References cited:
**EP-A-0 008 767**
**EP-A-0 056 488**
**WO-A-82/03854**
**BE-A- 622 053**
**DE-A-2 455 617**
**DE-A-2 726 978**
**DE-B-1 005 497**
**DE-B-1 203 254**
**FR-A-1 006 012**
**FR-A-1 303 231**
**FR-A-1 600 072**
**FR-A-2 370 023**
**FR-A-2 370 024**
**FR-A-2 418 025**
**GB-A-1 233 121**
**GB-A-1 277 242**

(73) Proprietor: **DAVY McKEE (LONDON) LIMITED**
**250, Euston Road**
**London NW1 2PG (GB)**

(72) Inventor: **BRADLEY, Michael William**
**52 Trefoil Wood Marton**
**Middlesbrough (GB)**
Inventor: **HARRIS, Norman**
**22 Grantham Road Norton**
**Cleveland (GB)**
Inventor: **TURNER, Keith**
**12 The Avenue Fairfield**
**Stockton-on-Tees Cleveland (GB)**

(74) Representative: **Eyles, Christopher Thomas et al**
**BATCHELLOR, KIRK & EYLES 2 Pear Tree Court**
**Farringdon Road**
**London, EC1R 0DS (GB)**

(56) References cited:
**GB-A-2 089 803**
**US-A-3 637 528**

**M. FREIFELDEN: "Catalytic hydrogenation organic synthesis", Ed. J. Wiley & Sons (1978), pages 129,130**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 104 197 B1

**Description**

This invention relates to the production of ethanol.

Ethanol is a major commercial bulk chemical. Although some ethanol for industrial purposes is produced by fermentation of sugar, starch and similar raw materials of biological origin, the feedstock predominantly used in most industrialised countries for production of ethanol is ethylene, which is catalytically hydrated by direct vapour phase reaction with water in the presence of a supported phosphoric acid catalyst.

Ethylene is a petroleum-based feedstock and so, with increasing depletion of the world's petroleum reserves there is interest in producing ethanol by processes that are ultimately based upon coal or natural gas as feedstock, rather than upon petroleum based feedstocks, such as ethylene.

Several processes have been described in the literature for conversion of carbonaceous feedstocks, such as coal and natural gas, to ethanol. The first stage in such procedures is usually the production of synthesis gas, i.e. a $CO:H_2$ mixture of appropriate composition, followed by methanol synthesis and then by further reactions such as reaction of methanol with further synthesis gas. Although methanol can be synthesised in high yield and with good selectivity from synthesis gas, the second stage of this route to ethanol has usually proceeded at best at about 80% selectivity to "realisable ethanol", based upon the methanol starting material (see, for example, GB—A—2053915). Moreover the conversion of methanol to ethanol has required the use of high temperatures and high pressures and results in production of significant quantities of by-products of relatively low value.

It is known that methanol can be carbonylated by reaction with carbon monoxide in the presence of a suitable catalyst to yield acetic acid. A typical process is described in GB—A—1277242, for example. Further documents describing reactions of this type are described hereafter.

It is also well known that acetic acid can be converted to an alkyl acetate.

Hydrogenolysis of esters to yield the corresponding alcohol or alcohols is also known. For example, "Catalytic Hydrogenation in Organic Synthesis", by Mr. Freifelden, published by Wiley in 1978, states at page 129 that barium promoted copper chromite is the catalyst of choice for this reaction, whilst conditions are rather drastic, use of a temperature of 250°C and a pressure of 200 to 250 bar being recommended.

Example 3 of DE—B—1005497 discloses a process in which a mixture of the methyl ester of coconut oil fatty acid and methanol is hydrogenated in the presence of pieces of a copper-zinc contact catalyst at a temperature of 240°C and 250 bar.

FR—A—1600072 teaches catalytic reduction of esters of dimeric or oligomeric acids to yield polyalcohols using a copper and/or zinc catalyst at a temperature of 150 to 350°C and at a pressure of 200 to 300 bar.

IN DE—B—1203254 there is described a process for the production of 1,4-dimethylolcyclohexane by catalytic hydrogenation of dimethyl hexahydroterephthalate using a catalyst containing copper and zinc at a temperature of 250°C and at a pressure of 250 bar.

FR—A—1303231 teaches use of copper-containing catalysts, including catalysts containing copper, zinc and chromium, for hydrogenolysis of esters such as a mixture of butyl esters of $C_{10}$ to $C_{20}$ acids or dibutyl at 250 bar and a temperature of 250°C.

FR—A—2418025 discloses in Example 13 hydrogenation of a solution of 47 to 52% of ethylene glycol glycolate in ethylene glycol at a pressure of 106.46 bar at temperatures of 204.4°C and 182.2°C, using a Cu-Zn and silica catalyst.

There is a need for a process capable of converting methanol to ethanol under conditions employing relatively low temperatures and pressures and at high selectivity.

The present invention accordingly seeks to provide an improved process for production of ethanol from methanol, and hence ultimately from synthesis gas, the source of which can be any carbonaceous material, such as coal or natural gas, besides petroleum based feedstocks such as naphtha or gas oil. It further seeks to provide a route to ethanol from synthesis gas which can be carried out in high yield and at high conversion rates whilst using relatively low temperatures and pressures.

According to the present invention there is provided a process for the production of ethanol which comprises reacting methanol with carbon monoxide under carbonylation conditions in a carbonylation zone in the presence of a carbonylation catalyst to form acetic acid, converting resulting acetic acid to an alkyl acetate, contacting a vaporous mixture comprising resulting alkyl acetate and hydrogen in a hydrogenolysis zone with a hydrogenolysis catalyst consisting essentially of a reduced mixture of copper oxide and zinc oxide at a temperature in the range of from about 150°C to about 240°C and at a pressure in the range of from about 5 kg/cm$^2$ absolute (about 491 kPa) up to about 50 kg/cm$^2$ absolute (about 4906 kPa), and recovering from the hydrogenolysis zone a hydrogenolysis product comprising ethanol.

The process of the invention may be operated batchwise or on a semi-continuous basis, but is preferably operated on a continuous basis.

The methanol used as starting material can be synthesised directly in known manner from synthesis gas, desirably an approximately 2:1 $H_2$: CO mixture, or a suitable $H_2:CO:CO_2$ mixture, the source of which may be any suitable carbonaceous feedstock, e.g. coal, natural gas, naphtha, gas oil or other petroleum fraction. The synthesis gas as formed may have an $H_2:CO$ molar ratio of, for example, from about 1:1 to

about 3:1. This ratio may be adjusted in known manner to give an approximately 2:1 $H_2$:CO molar ratio or the synthesis gas may be used as formed for synthesis of methanol after suitable purification.

The reactions involved in the process of the invention can be summarised thus:

(a) carbonylation —
$$CH_3OH + CO = CH_3COOH \quad (1);$$

(b) esterification, e.g. —
$$CH_3COOH + ROH = CH_3COOR = H_2O \quad (2);$$

(c) hydrogenolysis —
$$CH_3COOR = 2H_2 = CH_3CH_2OH = ROH \quad (3).$$

As the synthesis of methanol from synthesis gas involves the reaction of one mole of carbon monoxide with 2 moles of hydrogen over a suitable catalyst:

$$CO = 2H_2 = CH_3OH,$$

the overall series of reactions from synthesis gas to ethanol involves the use of 2 moles of CO and 4 moles of $H_2$:

$$CO = 4H_2 = CH_3CH_2OH = H_2O.$$

In the above equations R is a monovalent organic radical, typically an alkyl or cycloalkyl radical containing, for example, from 1 to about 12 carbon atoms. In one preferred process R is ethyl so that the acetate ester subjected to hydrogenolysis is ethyl acetate. Such a process has the advantage that ethanol is essentially the sole alcohol produced so that product recovery is greatly simplified. In cases where R is other than ethyl, for example where R is methyl, then the hydrogenolysis yields a mixture of ethanol and the alcohol of formula ROH, such as methanol, which can then be separated, e.g. by fractional distillation under normal, reduced, or elevated pressure to enable recovery of product ethanol as well as of the alcohol of formula ROH.

As examples of other acetate esters that can be used in the process of the invention there can be mentioned, for example, methyl acetate, *n*- and *iso*--propyl acetates, *n*-, *iso*- and *t*-butyl acetates, *n*-amyl acetate, *n*-hexyl acetate, 2-ethylhexyl acetate, cyclohexyl acetate, and the like.

In operation of the process it is preferred to recycle alcohol of formula ROH recovered from the hydrogenolysis step for esterification of further acetic acid. When R is ethyl, each mole of ethyl acetate that is hydrogenolysed yields essentially 2 moles of ethanol; part of the ethanol produced can be recycled for esterification of further acetic acid and production of further ethyl acetate, whilst the remainder of the ethanol produced by hydrogenolysis is passed forward for product recovery and possibly for further purification.

Carbonylation of methanol to form acetic acid is effected in the presence of an effective amount of an appropriate catalyst. Such catalysts are effective for catalysing the addition of carbon monoxide across the carbon-oxygen bond of methanol to form acetic acid. Examples of suitable catalysts include those containing Group VIII metals, such as cobalt, nickel, rhodium, and the like. Preferred catalyst systems include those comprising a rhodium containing component and a promoter component, such as iodine, bromine or a compound of bromine or iodine. As rhodium component there may be mentioned rhodium metal (in finely divided form), simple rhodium salts, organorhodium compounds, and coordination compounds of rhodium. Specific examples include:

Rh metal, $RhCl_3$, $RhBr_3$, $Rh_2O_3$,
$Rh(NO_3)_3 \cdot 2H_2O$, $Rh_2(CO)_8$, $RhCl_3 \cdot 3H_2O$, $RhBr_3$,
$Rh_2(CO)_4I_2$, $RhBr[(C_6H_5)_3P]_3$, $RhI\ [(C_6H_5)_3P]_3$,
$RhCl[(C_6H_5)_3P]_3$, $RhCl\ [(C_6H_5)_3P]_3H_2$, $[(C_6H_5)_3P]_3Rh\ (CO)H$,
$K_4Rh_2I_2(SnI_3)_4$, $Rh[(C_6H_5)_3P]_2(CO)I$, $RhCl[(C_6H_5)_3P]_2(CH_3I)_2$,
$Rh(CO)[P(C_6H_5)_3]_2Cl$, $Rh\ [(n\text{-}C_4H_9)_3P]_2(CO)I$,
$RhCl(CO)[(C_6H_5)_3As]_2$, and the like.

Promoters include compounds of the formula $RX_n$ wherein X is halogen, n is 1 to 3, and R is alkyl, alkylene or aryl (e.g. $CH_3I$, $C_6H_5Br$, $CH_3CH_2I$ and $ICH_2CH_2I$), as well as $I_2$, $I_3^-$, $Br_2$, $Br_3^-$, HBr, HI, RCOBr or RCOI, where R is alkyl or aryl (e.g. $CH_3COI$), and substituted and unsubstituted ammonium, phosphonium, or stibonium bromides or iodides (e.g. $NH_4I$, $NH_4I_3$, $PH_4I_3$, $PH_4Br_3$, and $(C_6H_5)_3PI_2$).

Typical carbonylation conditions include use of partial pressures of carbon monoxide in the range of from about 1 psig (0.07 kg/cm$^2$ gauge or 6.9 kPa gauge) to about 25,000 psig (1757.5 kg/cm$^2$ gauge or 172.5 MPa gauge), preferably in the range of from about 10 psig (0.7 kg/cm$^2$ gauge or 69 kPa gauge) to about 1000 psig (70.3 kg/cm$^2$ gauge or 6900 kPa gauge) and temperatures in the range of from about 50°C to about 300°C, preferably in the range of from about 100°C to about 240°C.

When using such rhodium catalysts there is produced in the carbonylation zone acetic acid, and possibly also methyl acetate as by-product. Such by-product methyl acetate can be recycled to the carbonylation zone and admixed with feed methanol so as to form a feed mixture containing methanol and methyl acetate in a ratio of not more than about 2:1, e.g. a feed mixture having a methanol:methyl acetate ratio of from about 0.001:1 or less up to about 2:1. If insufficient methyl acetate is produced in the carbonylation zone to provide the desired methanol:methyl acetate ratio, then part of the acetic acid can be esterified to give methyl acetate, which is recycled whilst the remaining acetic acid is passed on to form an acetate ester, such as ethyl acetate, for hydrogenolysis. Alternatively the crude acetic acid/methyl acetate product mixture can be passed forward to the esterification zone where the acetic acid is esterified to give, for example, ethyl acetate and the resulting methyl acetate/ethyl acetate mixture can then be hydrogenolysed to give a mixture of methanol and ethanol which is separated into methanol and ethanol streams, of which the methanol stream can be recycled to the carbonylation zone. In any event the conditions in the carbonylation zone are preferably selected to ensure at least about 90% conversion of methanol so as to maximise production of acetic acid. Carbonylation using the above-mentioned rhodium and promoter combination catalyst systems can be operated as a liquid phase process or as a vapour phase process. Further details can be obtained, for example, from GB—A—1233121. A similar process is described in GB—A—1277242.

Other carbonylation catalysts include nickel based catalysts, such as those described in US—A—4134912 and in US—A—4356320, as well as in GB—A—2089803. Such nickel-based catalysts are used in the presence of an iodide and include, for example:

1. Catalysts consisting essentially of nickel or a nickel compound and a promoter which is tin or tin compound (see US—A—4134912).

2. Catalysts consisting essentially of nickel or a nickel compound and a phosphine promoter (see US—A—4356320).

3. Catalysts comprising nickel or a nickel compound, a molybdenum or tungsten co-catalyst component, and a promoter comprising an organo-phosphorus compound or an organo-nitrogen compound wherein the phosphorus and nitrogen are trivalent (see GB—A—2089803).

When using a nickel-based carbonylation catalyst, the more volatile components such as alkyl iodide, e.g. methyl iodide, unreacted methanol, and by-products such as dimethyl ether and methyl acetate in the carbonylation mixture can be readily removed, for example by distillation, for recycling, and the net yield of product is substantially exclusively the desired acetic acid. In the case of liquid-phase reaction, which is preferred, the organic compounds are easily separated from the metal-containing components, for example by distillation. The reaction is suitably carried out in a reaction zone to which the carbon monoxide, methanol, the iodide, nickel or a nickel compound, the molybdenum or tungsten co-catalyst component (if used) and the promoter are fed.

In use of the nickel-based carbonylation catalysts a wide range of temperatures, e.g. 25 to 350°C are suitable but temperatures of 100 to 250°C are preferably employed and the more preferred temperatures generally lie in the range of 125 to 225°C. Typical residence times will generally fall in the range of 0.1 to 20 hours. The carbonylation reaction is carried out under super-atmospheric pressure but excessively high pressures, which require special high-pressure equipment, are not necessary. In general, the carbon monoxide partial pressure is preferably at least about 1.05 kg/cm$^2$ (103.5 kPa) but less than 141 kg/cm$^2$ (13800 kPa), particularly about 2.1 kg/cm$^2$ (207 kPa) to about 14.1 kg/cm$^2$ (1380 kPa), although CO partial pressures of about 0.07 kg/cm$^2$ (6.9 kPa) up to 703 kg/cm$^2$ (69000 kPa) can also be employed. The total pressure is, of course, that which will provide the desired carbon monoxide partial pressure to sustain a commercially acceptable reaction rate and preferably it is that required to maintain the liquid phase. The final carbonylation reaction mixture will normally contain volatile components such as hydrocarbyl iodide, unreacted methanol and may contain the corresponding methyl acetate and/or dimethyl ether along with the product acetic acid; these volatile components, after separation from the acetic acid, can be recycled to the reaction. At the end of the desired residence time the reaction mixture is separated into its several constituents, for example by distillation. Preferably, the reaction product is introduced into a distillation zone which may be a fractional distillation column, or a series of columns, effective to separate the volatile components from the acetic acid product and to separate the acetic acid product from the less volatile catalyst and promoter components of the reaction mixture. The boiling points of the volatile components are sufficiently far apart that their separation by conventional distillation presents no particular problem. Likewise, the higher-boiling organic components can be readily distilled away from the metal catalyst component or components and any organic promoter which may be in the form of a relatively non-volatile complex. The thus recovered metal catalyst component or components as well as promoter, including the iodide component, can then be combined with fresh amounts of methanol and carbon monoxide and reacted to produce additional quantities of acetic acid.

Although not necessary, the carbonylation step can be carried out in the presence of a solvent or diluent. The presence of a higher-boiling solvent or diluent, preferably the product acid itself, i.e. acetic acid, or the corresponding ester, i.e. methyl acetate, will make it possible to employ lower total pressures. Alternatively, the solvent or diluent may be any organic solvent which is inert in the environment of the process such as hydrocarbons, e.g. octane, benzene, toluene, xylene and tetralin, or halogenated hydrocarbons such as the chlorobenzenes, e.g. trichlorobenzene, or carboxylic acids, or esters such as

ethylene glycol ether monoacetates, and the like. Mixtures of solvents can also be used, such as mixtures of methyl acetate and acetic acid.

In the carbonylation step the carbon monoxide may be employed in substantially pure form, as available commercially, but inert diluents such as carbon dioxide, nitrogen, methane, and noble gases can be present if desired. The presence of inert diluents does not affect the carbonylation reaction but their presence makes it necessary to increase the total pressure in order to maintain the desired CO partial pressure. The presence of minor amounts of water such as may be found in the commercial forms of the reactants is, however, entirely acceptable. Hydrogen is not objectionable and even may tend to stabilise the nickel-based carbonylation catalysts. Indeed, in order to obtain low CO partial pressures the CO fed may be diluted with hydrogen or any inert gas such as those mentioned above. Surprisingly, the presence of hydrogen does not lead to the formation of reduction products. The diluent gas, e.g. hydrogen, may generally be used in amounts up to about 95%, if desired.

The metal catalyst components can be employed in any convenient form, viz., in the zero valent state or in any higher valent form. For example, the nickel and the tin, molybdenum or tungsten can be the metals themselves in finely divided form, or a compound, both organic or inorganic, which is effective to introduce the co-catalyst components into the reaction system. Thus, typical compounds include the carbonate, oxide, hydroxide, bromide, iodide, chloride, oxyhalide, hydride, lower alkoxide (methoxide), phenoxide or Sn, Mo, W or Ni carboxylates wherein the carboxylate ion is derived from an alkanoic acid of 1 to 20 carbon atoms such as acetates, butyrates, decanoates, laurates, benzoates, and the like. Similarly, complexes of any of the metal co-catalyst components can be employed, e.g. carbonyls and metal alkyls as well as chelates, association compounds and enol salts. Examples of other complexes include bis-(triphenylphosphine) nickel dicarbonyl, tricyclopentadienyl trinickel dicarbonyl tetrakis (triphenylphosphite) nickel, and corresponding complexes of the other components, such as molybdenum hexacarbonyl and tungsten hexacarbonyl. Included among the catalyst components listed above are complexes of the metal co-catalyst components with organic promoter ligands derived from the organic promoters hereinafter described.

Particularly preferred are the elemental forms of the metals, their halides, especially iodides, and their organic salts, e.g. acetates. It will be understood that the foregoing compounds and complexes are merely illustrative of suitable forms of the several co-catalyst components and are not intended to be limiting.

The tin promoter is preferably employed in elemental form or in the form of a halide, such as stannic iodide, stannous iodide, stannic chloride and stannic bromide, or a hydrocarbyl tin compound such as tetraphenyl tin, tetra *n*-butyl tin and dibutyl diphenyl tin, or an oxide such as stannous oxide and stannic oxide, or an organo oxide such as dimethyl tin oxide and diphenyl tin oxide, or a carboxylate such as stannous caproate and tri n-propyltin acetate, or an organo-halide such as dimethyl tin di-chloride and methyl tin trichloride. The most preferred tin compounds are the halides, the organo halides and the hydrocarbyl tins.

The specified metal catalyst components employed may contain impurities normally associated with the commercially available metal or metal compounds and need not be purified further.

The organo-phosphorus promoter is preferably a trivalent phosphorus compound, for example a phosphine of the formula

$$\begin{array}{l} R^1 \\ | \\ P{-}R^3 \\ | \\ R^2 \end{array}$$

wherein $R^1$, $R^2$ and $R^3$ may be the same or different, and are alkyl, cycloalkyl, or aryl groups, preferably containing 1 to 20 carbon atoms in the case of alkyl and cycloalkyl groups and 6 to 18 carbon atoms in the case of aryl groups. Other organo-phosphorus promoters include cyclic amides, such as hexamethylphosphoramide, and halophosphines. Typical phosphines include trimethylphosphine, tripropyl-phosphine, tricyclohexylphosphine and triphenylphosphine. Preferably the organo-nitrogen promoter is a tertiary amine or a polyfunctional nitrogen-containing compound, such as an amide, a hydroxy amine, a keto amine, a di-, tri and other polyamine or a nitrogen-containing compound which comprises two or more other functional groups. Typical organo-nitrogen promoters include 2-hydroxypyridine, 8-quinolinol, 1-methylpyrrolidone, 2-imidazolidone, N,N-dimethylacetamide, dicyclohexylacetamide, dicyclohexyl-methylamine, 2,6-diaminopyridine, 2-quinolinol, N,N-diethyltoluamide, and imidazole.

Although generally the organic promoter is added separately to the carbonylation catalyst system, it is also possible to add it as a complex with any of the co-catalyst metals, such as bis(triphenyl-phosphine) nickel dicarbonyl and tetrakis(triphenyl phosphite) nickel. Both free organic promoters and complexed promoters can also be used. When a complex of the organic promoter and the co-catalyst metal is used, free organic promoter can also be added.

The amount of each metal catalyst component employed is in no way critical and is that which will provide the desired suitable and reasonable reaction rate since reaction rate is influenced by the amount of catalyst. Typically, however, each catalyst component is employed in the amount of 1 mol per 100 to 10,000

mols of methanol, preferably 1 mol per 100 to 5,000 mols of methanol and most preferably 1 mol per 500 to 1,000 mols of methanol.

The ratio of nickel to the second metal co-catalyst component can vary. Typically, it is one mol of the nickel per 0.01 to 100 mols of the second metal co-catalyst component, preferably the nickel component is used in the amount of 1 mol per 0.1 to 20 mols, most preferably 1 mol per 1 to 10 mols of the second metal co-catalyst component.

The quantity of organic promoter can also vary widely but typically it is used in the amount of 1 mol per 0.1 to 10 mols of the nickel catalyst component, preferably 1 mol per 0.5 to 5 mols, most preferably 1 mole per 1 to 5 mols of the nickel catalyst component.

The amount of iodide component may also vary widely but, in general, it should be present in an amount of at least 10 mols (expressed as I) per hundred mols of methanol. Typically, there are used 10 to 50 mols of the iodide per 100 mols of methanol, preferably 17 to 35 moles per 100 moles. Ordinarily, more than 200 mols of iodide per 100 mols of methanol are not used. It will be understood, however, that the iodide component does not have to be added to the system as a hydrocarbyl iodide but may be supplied as another organic iodide or as the hydroiodide or other inorganic iodide, e.g. a salt, such as the alkali metal or other metal salt, or even as elemental iodine.

Preferably carbonylation is conducted in a continuous manner in which the reactants and catalyst are continuously supplied to the appropriate reaction zone and the reaction mixture continuously distilled to separate the volatile organic constituents and to provide a net product consisting essentially of acetic acid with the other organic components being recycled and, in a liquid-phase reaction a residual catalyst continuing fraction also being recycled.

It will also be apparent that the catalytic carbonylation reaction can be carried out in the vapour phase, if desired, by appropriate control of the total pressure in relation to the temperature so that the reactants are in vapour form when in contact with the carbonylation catalyst. In the case of vapour-phase operation, if desired, the carbonylation catalyst components may be supported i.e. they may be dispersed on a carrier of conventional type such as alumina, silica, silicon carbide, zirconia, carbon, bauxite, attapulgus clay, and the like. The catalyst components can be applied to the carriers in conventional manner, e.g. by impregnation of the carrier with a solution of the catalyst component. Concentrations upon the carrier may vary widely, e.g. 0.01 weight percent to 10 weight percent, or higher. Typical operating conditions for vapour-phase operation are a temperature of 100°C to 350°C., preferably 150°C to 275°C and most preferably 175°C to 255°C., a pressure of about 0.07 $kg/cm^2$ absolute (6.9 kPa absolute) to about 350 $kg/cm^2$ absolute (34500 kPa absolute), preferably about 3.5 $kg/cm^2$ absolute (345 kPa absolute) to about 105.5 kg/$cm^2$ absolute (10350 kPa absolute), and most preferably about 10.5 $kg/cm^2$ absolute (1035 kPa absolute) to about 35 $kg/cm^2$ absolute (3450 kPa absolute), with gas hourly space velocities of 50 to 10,000 $hr^{-1}$, preferably 200 to 6,000 $hr^{-1}$ and most preferably 500 to 4,000 $hr^{-1}$ (STP). In the case of a supported catalyst, the iodide component is included with the reactants and not on the support.

As noted above a mixture of CO and $H_2$ can be used in the carbonylation step. Hence it is possible to supply a synthesis gas, e.g. a 2:1 $H_2$:CO molar mixture to the carbonylation stage, and to recover the hydrogen subsequently for use in the hydrogenolysis step. Alternatively, as described further below, it is possible to supply to the carbonylation zone a CO-rich gas exiting the hydrogenation zone or a mixture thereof with make up synthesis gas.

In the ester formation step of a particularly preferred process acetic acid produced in the carbonylation step is esterified with an alcohol, preferably ethanol, in the presence of a suitable esterification catalyst. Conveniently such ethanol comprises product ethanol recycled following recovery subsequent to the hydrogenolysis step. Esterification can be carried out in the liquid phase or in the vapour phase. Preferably the esterification step is operated as a continuous process.

In liquid phase esterification an acidic esterification catalyst is preferably used. Examples of suitable acidic esterification catalysts include sulphuric acid, *p*-toluenesulphonic acid, and acidic ion exchange resins such as Amberlyst 15. (The word "Amberlyst" is a trade mark).

According to one preferred procedure acetic acid from the carbonylation zone is admixed with excess ethanol and with a catalytic amount, e.g. from about 0.1% to about 1% by weight of the reaction mixture, of sulphuric acid typically having a specific gravity of from about 1.53 to about 1.84 (about 50 to about 66 Bé). After allowing the mixture to equilibriate for a suitable period, e.g. about 5 minutes to about 3 hours, it is preheated to about 80°C and fed to the upper end of a distillation column, from which an ethanol/ethyl acetate/water mixture is recovered overhead, whilst sulphuric acid and water are removed as a bottom product. This distillation column is heated by introduction of live steam at its bottom end. The overhead mixture is fed to a recovery column from which, at a top temperature of 70°C, a ternary azeotrope (83% ethyl acetate, 9% ethanol, and 8% water) is recovered overhead. The resulting distillate is cooled and the organic layer which separates is redistilled to give a small amount of ternary azeotrope as overhead product, the bottom product comprising substantially pure ethyl acetate which is passed on to the hydrogenolysis step. Water and alcohol in excess of that required to form the ternary azeotrope is returned from the bottom end of the recovery column to the first mentioned distillation column.

Vapour phase esterification of acetic acid with alcohols, such as ethanol, can be effected by passage of a vaporous mixture thereof over a suitable catalyst, such as silica gel, zironium dioxide, activated charcoal,

potassium hydrogen sulphate, phosphoric acid-treated coal, calcium phosphate, or an acidic ion exchange resin, such as Amerlyst 15.

According to an alternative procedure acetic acid is esterified by reaction with an olefin to yield the corresponding acetate ester. In such a step it is preferred to use an acidic esterification catalyst, for example one of those listed above.

The hydrogenolysis of esters is disclosed in International Patent Publication No. WO82/03854 which was published after the priority date claimed in the present application. The entire disclosure of that International Patent Publication is herein incorporated by reference.

In the hydrogenolysis step the vaporous mixture to be contacted with the catalyst contains, in addition to the acetate ester, hydrogen either alone or in admixture with other gases (desirably gases inert to the ester and the catalyst). Hence the gaseous mixtures containing hydrogen may include inert gases such as nitrogen, or carbon monoxide.

In view of the well known behaviour of carbon monoxide as a hydrogenation catalyst poison or inhibitor it is surprising that carbon monoxide supplied in the form of synthesis gas does not appear to poison the copper-containing hydrogenolysis catalysts used in the present invention, even when present in gross amounts of about 10% or more by volume.

Thus the make up gas supplied to the hydrogenolysis zone may contain at least about 1% CO, and more usually at least about 10% by volume of carbon monoxide. Suitably it comprises a synthesis gas produced by partial oxidation of a suitable hydrocarbon feedstock, such as methane, natural gas, a petroleum gas, such as propane or butane, or a petroleum fraction, such as naphtha, a gas oil, or a fuel oil, or by catalytic steam reforming of a suitable hydrocarbon feedstock such as methane, natural gas, propane, butane, or naphtha, or by reaction of steam/$O_2$ mixtures with coal. It is preferred to use a synthesis gas that contains CO and $H_2$ in a molar ratio in the range of from about 1:1.5 to about 1:2.5, especially about 1:2. If the synthesis gas as produced, either by partial oxidation or by steam reforming, has a CO:$H_2$ molar ratio that differs greatly from this range, then the CO:$H_2$ ratio is preferably adjusted by known methods, e.g. subjection to a water gas shift reaction followed by $CO_2$ removal, if and as necessary, to a value within this range. A gas with an approximately 1:2 CO:$H_2$ molar ratio is preferably used.

Prior to its introduction to the hydrogenolysis zone steps are desirably taken to ensure that the synthesis gas is substantially free from sulphurous impurities. When using steam reforming to generate the synthesis gas from a hydrocarbon feedstock containing sulphurous impurities it is best to desulphurise the feedstock prior to steam reforming. Similarly when using partial oxidation of gaseous or liquid hydrocarbon feedstocks, such as methane or naphtha, containing sulphurous impurities, it will often be convenient to desulphurise the feedstock prior to oxidation thereof by contact with a suitable desulphurisation medium, such as activated alumina or zinc oxide, although it is also possible to effect desulphurisation after partial oxidation by contacting the synthesis gas with a bed of desulphurisation medium such as zinc oxide, possibly after removing the bulk of the sulphur-containing compounds by any of the well known sulphur removal processes (e.g. Rectisol process, diethanolamine wash process etc.).

The hydrogenolysis zone may take the form of one or more externally cooled tubular reactors. Alternatively the hydrogenolysis zone may comprise one or more adiabatic reactors arranged in series.

The hydrogenolysis step is conducted at a temperature of between about 75°C and about 300°C; although in many cases the temperature may lie in the range of from about 150°C to about 200°C, in most cases it typically is between about 180°C and about 240°C. The total pressure is between about 0.1 kg/cm$^2$ absolute (about 9.8 kPa) and about 100 kg/cm$^2$ absolute (about 8913 kPa), preferably not more than about 50 kg/cm$^2$ absolute (about 4906 kPa), and even more preferably between about 5 kg/cm$^2$ absolute (about 491 kPa) and about 25 kg/cm$^2$ absolute (about 2453 kPa).

The mixture of CuO and ZnO, before reduction, preferably contains from about 5 to about 95 percent by weight, typically from about 10 to about 70 percent by weight, of CuO and from about 95 to about 5 percent by weight, typically from about 90 to about 30 percent by weight, of ZnO. Hence the mixture may contain, for example, from about 20 to about 40 percent by weight of CuO and from about 60 to about 80 percent by weight of ZnO. A preferred mixture, for example, comprises from about 30 to about 36 percent by weight of CuO and from about 62 to about 68 percent by weight of ZnO. Other particularly preferred mixtures comprise from about 65 to about 85 percent by weight of CuO and from about 35 to about 15 percent by weight of ZnO, for example mixtures comprising from about 68 to about 75 percent by weight of CuO and from about 32 to about 25 percent by weight of ZnO. The hydrogenolysis catalyst may contain minor amounts of other materials such as carbon, sodium, titanium, zirconium, manganese, silica, diatomaceous earth, kieselguhr, and aluminium oxide. Such other materials do not usually comprise more than about 20 percent by weight calculated (except in the case of carbon) as oxide. In the case of sodium it is best not to exceed about 0.5 percent by weight, calculated as oxide. Hence other preferred catalysts include mixtures comprising from about 40 to about 50 weight percent each of CuO and ZnO and from 0 to about 20 weight percent of alumina. The catalyst is, however, preferably essentially free from other metals, particularly from metals of Group VIII of the Periodic Table, such as Fe, Co, Ni, Ru, Rh, Pd, Os, Ir, and Pt, as well as from Group VIB metals, such as Cr, Mo, and W, from the metals Ag, Re, Au and Cd, and also from elements of atomic number 80 and above, e.g. Hg and Pb. By the term "essentially free" we mean that the catalyst contains not more than about 0.1 wt% (i.e. not more than about 1000 ppm), and preferably not more than about 250 ppm, of the element in question. The catalyst may be prepared by any of the methods known in

the art of forming a composite of copper oxide and zinc oxide. The catalyst may be prepared by fixing the separate oxides, by coprecipitation of the oxalates, nitrates, carbonates, or acetates, followed by calcination. The coprecipitation method is preferred. Generally, the mixture of CuO and ZnO is reduced by hydrogen or carbon monoxide at a temperature in the range of between about 160°C and about 250°C for several hours, preferably for 8 to 24 hours, prior to contact with the vaporous mixture containing acetate ester and hydrogen. If the catalyst is charged in a pre-reduced form the period required for reduction can be reduced accordingly.

The mixture of CuO and ZnO is reduced prior to its use as catalyst in the hydrogenolysis step. Hydrogen or CO, or mixtures thereof, are generally mixed with a diluent gas such as steam, nitrogen, or combustion gas, to maintain the catalyst bed temperature and to carry away the heat of reduction.

Reduction of the mixture of CuO and ZnO is complete when no more hydrogen is being reacted as shown by analysis of the inlet and outlet hydrogen. Complete reduction of the mixture occurs when the total amount of water produced in the reduction is equal to the stoichiometric value of water which should be produced when a given amount of copper oxide is reduced to copper. This value is about 0.079 kg of water per kg of catalyst for a mixture containing 35 weight percent of CuO.

An inert carrier material may be included in the hydrogenolysis catalyst composition. The catalyst is generally formed into pellets, tablets, or any other suitable shape prior to use, by conventional techniques.

It is advantageous that the mixture of CuO and ZnO have an internal surface area of from about 25 to about 50 sq.m. per gram. The internal surface area may be determined by the well-known BET method.

The reaction product mixture from the hydrogenolysis step may be separated from any excess hydrogen by condensation and the excess hydrogen can be compressed and recycled. This reaction product mixture comprises, when the acetate ester is ethyl acetate, ethanol or, when another acetate ester is hydrogenolysed, a mixture of ethanol and another alcohol, i.e. the alcohol derived from the alcohol moiety of the acetate ester; in addition the reaction product mixture may comprise, possibly a minor amount of unconverted acetate ester. This mixture is separated in any suitable manner, e.g. by distillation. The ethanol may be used as recovered or it can be further purified in a conventional manner such as by fractional distillation. Preferably any unconverted acetate ester recovered is recycled to the hydrogenolysis stage. When ethyl acetate is used as the acetate ester, part of the ethanol recovered may be recycled to the esterification stage; when another acetate ester, such as methyl acetate is used, the other alcohol derived from the alcohol moiety of the acetate ester (e.g. methanol) may be recycled to the esterification stage.

In the hydrogenolysis step the partial pressure of the acetate ester may vary within wide limits, e.g. from about 0.05 kg/cm$^2$ (about 4.9 kPa) or less up to about 10 kg/cm$^2$ (about 981 kPa) or more. Care must however be taken to ensure that at all times the temperature of the vaporous mixture in contact with the hydrogenolysis catalyst is above the dew point of the acetate ester under the prevailing pressure conditions.

The vaporous mixture preferably contains at least an amount of hydrogen corresponding to the stoichiometric quantity of hydrogen required for hydrogenolysis. Usually an excess of hydrogen over the stoichiometric quantity will be present. In this case the excess hydrogen remaining after product recovery can be recycled to the hydrogenolysis zone. As will be apparent from equation (3) above, 2 moles of hydrogen are required for hydrogenolysis of the acetate ester.

The hydrogen:ester molar ratio within the vaporous mixture may vary within wide limits, from about 2:1 to about 100:1 or more.

As mentioned above, hydrogenolysis can be carried out using a gaseous mixture containing both CO and $H_2$. In this case the concentration of CO in the gas passing into the hydrogenolysis zone may range from about 1% by volume up to about 90% by volume or more. Usually it will be at least about 10% by volume and will often lie in the range of from about 10% by volume up to about 60% by volume, e.g. about 20% by volume to about 40% by volume. The make up gas supplied to the hydrogenolysis zone may thus be a synthesis gas, preferably one with a CO:$H_2$ molar ratio of about 1:2. Alternatively the make up gas supplied to the hydrogenolysis zone may be an off gas from the carbonylation zone or a mixture thereof with synthesis gas.

Hence, in a preferred process according to the invention, the carbonylation and hydrogenolysis steps are carried out using a circulating gas containing both carbon monoxide and hydrogen and make up gas for the circulating gas is supplied in the form of synthesis gas at a convenient point in the gas circuit. Thus the make up synthesis gas can be admixed with the off gas from the carbonylation zone and the resulting mixture supplied to the hydrogenolysis zone. Alternatively make up synthesis gas can be admixed with off gas from the hydrogenolysis zone and supplied to the carbonylation zone. Either or both of the carbonylation and hydrogenolysis zones may employ internal recirculation of gas. The level of inerts (e.g. $CH_4$, $N_2$, A, etc.) may be controlled by taking a purge stream from the circulating gas stream at some convenient point.

If a synthesis gas other than a 2:1 $H_2$:CO molar mixture is used, e.g. a 3:1 $H_2$CO molar mixture synthesis gas resulting from steam reforming of methane, then a pressure swing absorption unit can be included in the gas circulation loop in order to recover the excess $H_2$ for export beyond battery limits or for use as a fuel.

In order that the invention may be clearly understood and readily carried into effect some preferred forms of ethanol synthesis plant operating according to the process of the invention will now be described,

by way of example only, with reference to the accompanying diagrammatic drawings, Figures 1 to 3 of which are each a simplified flow sheet of the respective plant.

Referring to Figure 1 of the drawings, a synthesis gas plant 1 of conventional design is arranged to deliver a synthesis gas of suitable composition, desirably an approximately 2:1 $H_2$:CO molar mixture, by way of line 2 to a methanol synthesis plant 3, also of conventional design. Product methanol is passed by way of line 4 to carbonylation stage 5 in which it is reacted with carbon monoxide, supplied in the form of synthesis gas, by way of line 6. Carbonylation stage 5 may operate, for example, according to the teachings of the afore-mentioned British Patent Specification No. 1233121 or according to the teachings of United States Patent Specification No. 4134912 or 4356320 or British Patent Specification No. 2089803A. A gaseous stream comprising hydrogen and any unreacted carbon monoxide is passed by way of line 7 to hydrogenolysis zone 8, whilst product acetic acid (containing possibly minor amounts of methyl acetate) produced in carbonylation zone 5 is passed to esterification zone 9 by way of line 10.

In esterification zone 9 acetic acid supplied in line 10 is esterified with ethanol recycled in line 11. Esterification can be effected in zone 9 as a liquid phase process using as catalyst an esterification catalyst such as sulphuric acid or Amberlyst 15. Alternatively esterification can be carried out as a vapour phase process usin one of the catalysts listed above as suitable for such vapour phase esterification. Water produced in esterification zone 9 is removed in line 18.

Hydrogenolysis zone 8 contains a charge of a catalyst comprising a reduced mixture of CuO and ZnO, e.g. a catalyst of composition, before reduction, 30 to 36% by weight CuO and 62 to 68% by weight ZnO. This is supplied with a vaporous mixture comprising hydrogen supplied by way of line 7 and ethyl acetate supplied from esterification zone in line 12. Hydrogenolysis zone 8 may comprise a multi-tubular reactor or a single or multi-stage adiabatic reactor. The temperature in hydrogenolysis zone 8 may range from about 75°C to about 300°C and the pressure may range from about 0.1 $kg/cm^2$ absolute (about 6.9 kPa) up to about 50 $kg/cm^2$ absolute (about 4906 kPa). Ethanol (together possibly with a minor amount of methanol and with unchanged ethyl and/or methyl acetate) is recovered from hydrogenolysis zone 8 in line 13 and is separated by fractional distillation in product recovery zone 14 to give ethanol, which is recovered in line 15, methanol, which is recycled to carbonylation zone 5 in line 16, and methyl and/or ethyl acetate which is or are recycled to hydrogenolysis zone 8 in line 17. Ethanol for esterification of acetic acid in esterification zone 9 is recycled from line 15 in line 11.

In the plant of Figure 2 methanol is supplied in line 101 from a conventional methanol synthesis plant (not shown) to a carbonylation zone 102, which is generally similar to zone 5 of the plant of Figure 1. Zone 102 is also supplied with a CO-rich gas in line 103.

Acetic acid product from zone 102, together with any by-product methyl acetate, is passed by way of line 104 to esterification zone 105 to which is fed ethanol in line 106. Water produced as a result of the esterification reaction is recovered in line 107, whilst ethyl acetate is passed in line 108, to ester hydrogenolysis zone 109, which is generally similar to zone 8 of Figure 1. Hydrogenolysis zone 109 may contain, for example, a charge of a hydrogenolysis catalyst comprising about 70 to about 72% by weight copper oxide and about 18 to about 2% by weight zinc oxide on a suitable support. Hydrogen for the hydrogenolysis step is provided in the form of a mixture of recycled gas from carbonylation zone 102 in line 110 and make up synthesis gas supplied in line 111. This make up synthesis gas comprises an approximately 2:1 $H_2$:CO molar mixture and may be generated for example by subjection of methane to partial oxidation:

$$CH_4 + \tfrac{1}{2}O_2 = CO + 2H_2.$$

Alternatively a hydrocarbon feedstock such as naphtha, can be subjected to catalytic steam reforming:

$$-(CH_2)_n- + nH_2O = nCO + 2nH_2.$$

The same synthesis gas plant can be used to supply the synthesis gas in line 111 as is used to supply synthesis gas to the plant used to produce the methanol in line 101. Wihin zone 109 there may be internal recycle of gas. This mixture is fed to ester hydrogenation zone by means of compressor 112 and line 113. A purge gas stream is taken in line 114 in order to control the level of inerts (e.g. $N_2$, A, $CH_4$ etc.) in the circulating gas.

Ethanol product from the hydrogenolysis zone, together possibly with small amounts of ethyl acetate and methyl acetate is supplied by way of line 114 to a separation/purification zone 115. This typically comprises a fractional distillation arrangement. Ethanol product is recovered in line 116. Any methanol present is recovered in line 117. Ethanol required for esterification is recycled in line 106 to esterification zone 105. Any methyl acetate can be recycled in line 110.

As described, the alcohol of formula ROH used in the plant of Figure 2 is ethanol. If another alcohol, e.g. methanol or *i*-propanol, is used as the alcohol of formula ROH, then such alcohol ROH will be recycled in line 106 and the ester in line 108 will be the ester, $CH_3COOR$. Line 114 will then contain a mixture of ethanol and the alcohol ROH which is separated in zone 116.

It will be appreciated that as the drawings are diagrammatic many features which would be present in

an operating plant have not been described. For example, there may be provision for internal recycle of CO-rich gas within carbonylation zone 102.

In the plant of Figure 3, methane is supplied by way of line 201 to a steam reforming plant 202 of conventional design, which is also supplied with steam in line 203. Approximately 50% of the resulting approximately 3:1 $H_2$:CO molar mixture synthesis gas is fed by way of line 204 to a methanol synthesis plant 205, also of conventional design. Product methanol is fed in line 206 to a carbonylation/esterification plant 207 in which it is first carbonylated, by reaction with CO in a gaseous mixture containing both CO and $H_2$ supplied in line 208, to give acetic acid and then esterified by reaction with methanol supplied in line 209 to form methyl acetate. Carbonylation can be effected according to the teachings of British Patent Specification No. 1233121 or 2089803A or of United States Patent Specification No. 4134912 or 4356320. The resulting methyl acetate is passed in line 210 to a hydrogenolysis zone 211. Water produced in the esterification reaction is removed in line 212, whilst a CO-depleted gas is passed in line 213 to a pressure swing absorption plant 214 which is used to control the CO:$H_2$ ratio in the gas supplied in line 208. A hydrogen stream is recovered from plant 214 in line 215. Off gas from methanol synthesis plant 205 is introduced into the circulating gas by means of line 216. In hydrogenolysis plant 211 methyl acetate is subjected to hydrogenolysis, using as the source make up gas, the remainder of the synthesis gas from plant 202 which is supplied in line 217. The catalyst is that used in the plants of Figures 1 and 2. Ethanol is recovered, after suitable separation e.g. by fractional distillation, in line 218 whilst methanol is recycled in line 209. The off gas from hydrogenolysis plant 211 is supplied to pressure swing absorption plant 214 in line 219.

The invention is further illustrated in the following Examples.

Example 1

A. A solid supported catalyst containing a rhodium component dispersed upon an inert support is prepared in the following manner: 2.37 grams of rhodium chloride trihydrate having the formula, $RhCl_3 . 3H_2O$ is dissolved in 50 ml of water as solvent. The resulting solution is added to 30 g of a support consisting of 12—30 mesh high surface area carbon.

The mixture is dried at 25°C in air for 8 to 16 hours and then vacuum dried at 110°C for 8 to 16 hours.

A batch reactor is charged with the following ingredients: 0.396 grams of a rhodium compound having the formula $RhCl_3 . 3H_2O$, 28.8 grams of a promoter consisting of methyl iodide, $CH_3I$, 196.9 grams of acetic acid as a solvent, and 79 grams of methanol as feedstock.

The reactor is pressurised with carbon monoxide to a toal pressure of 70.3 kg/cm$^2$ gauge (6900 kPa gauge) corresponding to a carbon monoxide partial pressure of about 56.25 kg/cm$^2$ gauge (5520 kPa gauge) at the reaction temperature of 175°C. The reaction is carried out at a constant pressure to yield a solution containing the following distribution of products:

89.0 wt% Acetic Acid
3.6 wt% Methyl Iodide
8.4 wt% (Catalyst, etc.)

The selectivity to the formation of the desired acetic acid product is greater than 95% at substantially 100% conversion of methanol. No substantial amounts of byproducts such as aldehydes, dimethyl ether, higher boiling carboxylic acids, methane, or carbon dioxide are formed. The time required for 50% of the methanol to be converted to acetic acid is 335 minutes.

B. 58g ethanol, 228g acetic acid and 3g concentrated sulphuric acid are heated under reflux for 6 hours. The reaction mixture is then fractionated and the crude ester fraction is then washed with a little water, saturated with salt, washed with saturated sodium bicarbonate solution, saturated with salt, and dried over anhydrous sodium and magnesium sulphate, prior to redistillation. Ethyl acetate b.p. 76—77°C is obtained.

C. Ethyl acetate is pumped at a rate of 34.8 ml/hr to an electrically heated gas/liquid mixing device to which hydrogen was also supplied at a controlled rate and pressure. The resulting vaporous mixture is passed through a lagged, electrically heated line to a pre-heating coil prior to passage through a tubular reactor packed with 146 ml of a powdered catalyst. Both the tubular reactor and the pre-heating coil are immersed in a molten salt bath which was heated to 203°C. The vaporous mixture exiting the reactor is passed through a water cooled condenser and the resulting condensate is collected in a water-cooled knock out pot. The exit gas pressure is controlled to 10.55 kg/cm$^2$ absolute (1035 kPa). The non-condensed gases are then passed through a let-down valve, the gas flow rate being monitored downstream from this valve in a wet gas meter. A gas flow rate of 160.4 litres/hr (measured at atmospheric pressure) is maintained throughout the experiment.

The liquid condensate is analysed by gas chromatography using a 2 metre stainless steel column (6 mm outside diameter) packed with polyethylene glycol (nominal molecular weight 20,000) on Chromosorb PAW, a helium gas flow rate of 30 ml/minute and a flame ionisation detector. The instrument is fitted with a chart recorder having a peak integrator. The condensate is shown to contain both ethanol and ethyl acetate corresponding to a 86.2% conversion with essentially 100% selectivity to ethanol.

The catalyst used is charged to the reactor as a co-precipitated mixture of CuO and ZnO containing 33 ± 3% by weight CuO and 65 ± 3% by weight ZnO having a particle size in the range of 1.2 mm to 2.4 mm and an internal surface area of about 45 sq. m. per gram. This is pre-reduced in the reactor using a 5 vol% $H_2$ in $N_2$ gas mixture at 200°C for 17 hours followed by pure hydrogen at 200°C for 8 hours, the gas

flow rate in each case being about 20 litres/hr (measured at atmospheric pressure using the wet gas meter) and the gas pressure being 10.55 kg/cm$^2$ absolute (1035 kPa). After this pre-reduction stage the catalyst is at all times maintained in a hydrogen-containing atmosphere.

Example 2

A magnetically-stirred, glass-lined bomb is charged with 20 g methanol, 15 g of methyl acetate, 100 g of methyl iodide, 100 g of bis-triphenylphosphine nickel dicarbonyl plus 3 g of molybdenum hexacarbonyl as co-catalyst, and 3 g of triphenylphosphine, is swept out with argon and is pressured to 34.6 kg/cm$^2$ absolute (3400 kPa) with carbon monoxide. The vessel is heated to 185°C with stirring and the temperature is maintained at 185°C. After 1 hour reaction time, gas chromatographic analysis of the reaction effluent shows it to contain 5 mol% methyl acetate in excess of the charged amount and 87 mol% acetic acid. This represents a 92% conversion of methanol to the acetyl group.

B. Ethyl acetate is prepared by a similar method to that used in Part B of Example 1.

C. Using a similar procedure to that of Part C of Example 1, ethyl acetate is subjected to hydrogenolysis using a 1:1 $CO:H_2$ mixture in place of the hydrogen stream of Example 1. At an ethyl acetate flow rate of 292 ml/hr (i.e. a liquid hourly space velocity of 2.0 hr$^{-1}$) and a bath temperature of 198°C, conversion of ethyl acetate to ethanol is 25.6%, the selectivity to ethanol being essentially 100%.

In a comparison run using a 1:1 $N_2:H_2$ molar mixture in place of the 1:1 $CO:H_2$ molar mixture, the observed conversion to ethanol is 46.2%.

Example 3

The general procedure of Step C of Example 2 is repeated using methyl acetate in place of ethyl acetate. Using a 1:1 $CO:H_2$ molar mixture at a catalyst temperature of 180°C and a liquid hourly space velocity of 1.0 hr$^{-1}$, the observed conversion of methyl acetate to a mixture of methanol and ethanol is 17.7%.

In a comparison run under otherwise identical conditions, the observed conversion of methyl acetate is 35.2% when a 1:1 $N_2:H_2$ molar mixture replaces the 1:1 $CO:H_2$ molar mixture.

Example 4

The general procedure of Step C of Example 2 is repeated using a 20 ml sample of a pelleted catalyst (3 mm × 3 mm) comprising 71.5% by weight CuO and 18.5% ZnO on an inert support heated by means of a fluidised sand bath to 185°C. At an ethyl acetate flow rate of 27.2 ml/hr and using a mixture of 62.4 litres/hr $H_2$ and 31.3 litres/hr CO the conversion to ethanol is 22.1% at essentially 100% selectivity. In a comparison run under the same conditions except that the gas mixture is 62.4 litres/hr $H_2$ and 31.3 litres/hr $N_2$ the conversion to ethanol is 56.0%, also at essentially 100% selectivity.

**Claims**

1. A process for the production of ethanol which comprises reacting methanol with carbon monoxide under carbonylation conditions in a carbonylation zone in the presence of a carbonylation catalyst to form acetic acid, converting resulting acetic acid to an alkyl acetate, contacting a vaporous mixture comprising resulting alkyl acetate and hydrogen in a hydrogenolysis zone with a hydrogenolysis catalyst consisting essentially of a reduced mixture of copper oxide and zinc oxide at a temperature in the range of from about 150°C to about 240°C and at a pressure in the range of from about 5 kg/cm$^2$ absolute (about 491 kPa) up to about 50 kg/cm$^2$ absolute (about 4906 kPa), and recovering from the hydrogenolysis zone a hydrogenolysis product comprising ethanol.

2. A process according to claim 1, characterised in that the acetate ester is ethyl acetate and part of the ethanol recovered from the hydrogenolysis zone is recycled to the esterification step.

3. A process according to claim 1 or claim 2, characterised in that the pressure in the hydrogenolysis step is in the range of from about 5 kg/cm$^2$ absolute (about 491 kPa) to about 25 kg/cm$^2$ absolute (about 2453 kPa).

4. A process according to any one of claims 1 to 3, characterised in that the temperature in the hydrogenolysis step is in the range of from about 180°C to about 240°C.

5. A process according to any one of claims 1 to 4, characterised in that the hydrogenolysis catalyst comprises a reduced mixture of copper oxide and zinc oxide derived from a mixture comprising, before reduction, from about 10 to about 70 percent by weight CuO and about 90 to about 30 percent by weight ZnO.

6. A process according to claim 5, characterised in that the mixture comprises from about 20 to about 40 percent by weight CuO and from about 60 to 80 percent by weight ZnO.

7. A process according to any one of claims 1 to 6, characterised in that hydrogenolysis is effected with a gas mixture containing hydrogen and at least about 1% by volume of carbon monoxide.

8. A process according to any one of claims 1 to 7, characterised in that carbonylation and hydrogenolysis are effected using a circulating gas containing both hydrogen and carbon monoxide, and synthesis gas is supplied as make up gas to the circulating gas.

**Patentansprüche**

1. Verfahren zur Herstellung von Äthanol, bei dem man Methanol mit Kohlenmonoxid in einer Carbonylierungszone unter Carbonylierungsbedingungen in Gegenwart eines Carbonylierungskatalysators zu Essigsäure umsetzt, die entstandene Essigsäure zu einem Alkylacetat umsetzt, ein dampfförmiges Gemisch aus dem entstandenen Alkylacetate und Wasserstoff in einer Hydrogenolysezone mit einem im wesentlichen aus einem reduzierten Gemisch aus Kupferoxid und Zinkoxid bestehenden Hydrogenolysekatalysator bei einer Temperatur in dem Bereich von etwa 150°C bis etwa 240°C und einem Druck in dem Bereich von etwa 5 $kg/cm^2$ absolut (etwa 491 kPa) bis zu etwa 50 $kg/cm^2$ absolut (etwa 4906 kPa) in Berührung bringt und aus der Hydrogenolysezone ein Äthanol aufweisen des Hydrogenolyseprodukt gewinnt.

2. Verfahren nach Anspruch 1, dadurch gekennzeichnet, daß der Essigsäureester Äthylacetat ist und ein Teil des aus der Hydrogenolysezone gewonnenen Äthanols zur Veresterungsstufe zurückgeführt wird.

3. Verfahren nach Anspruch 1 oder 2, dadurch gekennzeichnet, daß der Druck in der Hydrogenolysestufe in dem Bereich von etwa 5 $kg/cm^2$ absolut (etwa 491 kPa) bis etwa 25 $kg/cm^2$ absolut (etwa 2453 kPa) liegt.

4. Verfahren nach einem der Ansprüche 1 bis 3, dadurch gekennzeichnet, daß die Temperatur in der Hydrogenolysestufe in dem Bereich von etwa 180°C bis etwa 240°C liegt.

5. Verfahren nach einem der Ansprüche 1 bis 4, dadurch gekennzeichnet, daß der Hydrogenolysekatalysator ein reduziertes Gemisch aus Kupferoxid und Zinkoxid aufweist, das sich aus einem Gemisch vor der Reduktion von etwa 10 bis etwa 70 Gew.-% CuO und etwa 90 bis etwa 30 Gew.-% ZnO ableitet.

6. Verfahren nach Anspruch 5, dadurch gekennzeichnet, daß das Gemisch etwa 20 bis etwa 40 Gew.-% CuO und etwa 60 bis 80 Gew.-% ZnO aufweist.

7. Verfahren nach einem der Ansprüche 1 bis 6, dadurch gekennzeichnet, daß man die Hydrogenolyse mit einen Gasgemisch durchführt, das Wasserstoff und wenigstens, etwa 1 Vol.-% Kohlenmonoxid enthält.

8. Verfahren nach einem der Ansprüche 1 bis 7, dadurch gekennzeichnet, daß man die Carbonylierung und Hydrogenolyse unter Verwendung eines Wasserstoff und Kohlenmonoxid enthaltenden, zirkulierenden Gases durchführt und dem zirkulierenden Gas als Ergänzungsgas Synthesegas zuführt.

**Revendications**

1. Procédé pour la production d'éthanol qui comprend la réaction du méthanol avec du monoxyde de carbone dans des conditions de carbonylation dans une zone de carbonylation en présence d'un catalyseur de carbonylation pour former de l'acide acétique, la conversion de l'acide acétique obtenu en un acétate d'alkyle, le contact d'un mélange à l'état de vapeur comprenant l'acétate d'alkyle obtenu et de l'hydrogène dans une zone d'hydrogénolyse avec un catalyseur d'hydrogénolyse constitué essentiellement d'un mélange réduit d'oxyde de cuivre et d'oxyde de zinc à une température dans la gamme d'environ 150°C à environ 240°C et sous une pression dans la gamme d'environ 5 $kg/cm^2$ absolus (environ 491 kpa) à environ 50 $kg/cm^2$ absolus (environ 4906 kpa) et la récupération à partir de la zone d'hydrogénolyse d'un produit d'hydrogénolyse comprenant de l'éthanol.

2. Procédé selon la revendication 1, caractérisé en ce que l'ester acétique est l'acétate d'éthyle et une partie de l'éthanol récupéré dans la zone d'hydrogénolyse est recyclée dans le stade d'estérification.

3. Procédé selon la revendication 1 ou la revendication 2, caractérisé en ce que la pression dans le stade d'hydrogénolyse est dans la gamme d'environ 5 $kg/cm^2$ absolus (environ 491 kpa) à environ 25 $kg/cm^2$ absolus (environ 2453 kpa).

4. Procédé selon l'une quelconque des revendications 1 à 3, caractérisé en ce que la température dans le stade d'hydrogénolyse est dans la gamme d'environ 180°C à environ 240°C.

5. Procédé selon l'une quelconque des revendications 1 à 4, caractérisé en ce que le catalyseur d'hydrogénolyse comprend un mélange réduit d'oxyde de cuivre et d'oxyde de zinc dérivant d'un mélange comprenant, avant réduction, d'environ 10 à environ 70% en poids de CuO et d'environ 90 à environ 30% en poids de ZnO.

6. Procédé selon la revendication 5, caractérisé en ce que le mélange comprend environ 20 à environ 40% en poids de CuO et d'environ 60 à 80% en poids de ZnO.

7. Procédé selon l'une quelconque des revendications 1 à 6, caractérisé en ce que l'hydrogénolyse est effectuée avec un mélange gazeux contenant de l'hydrogène et au moins environ 1% en volume de monoxyde de carbone.

8. Procédé selon l'une quelconque des revendications 1 à 7, caractérisé en ce que la carbonylation et l'hydrogénolyse sont effectuées par emploi d'un gaz circulant contenant à la fois de l'hydrogène et du monoxyde de carbone, et du gaz de synthèse est fourni comme gaz d'appoint au gaz circulant.

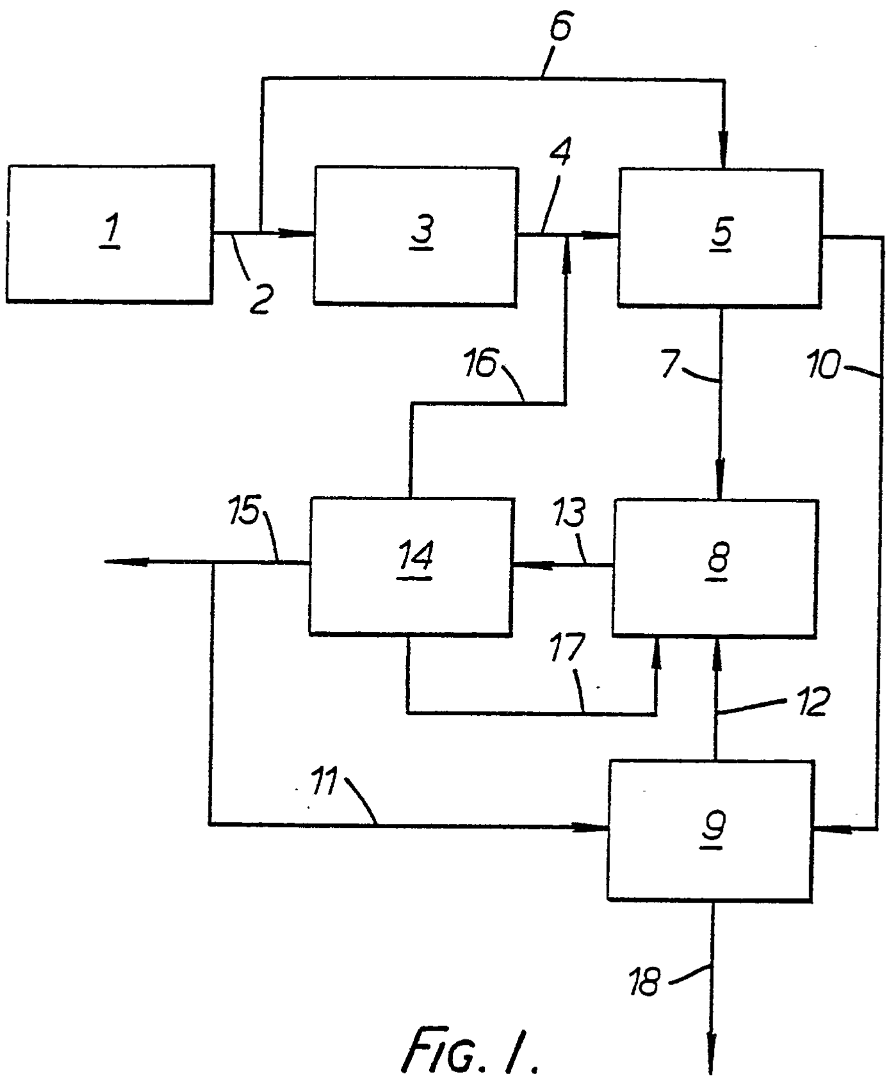


FIG. 1.

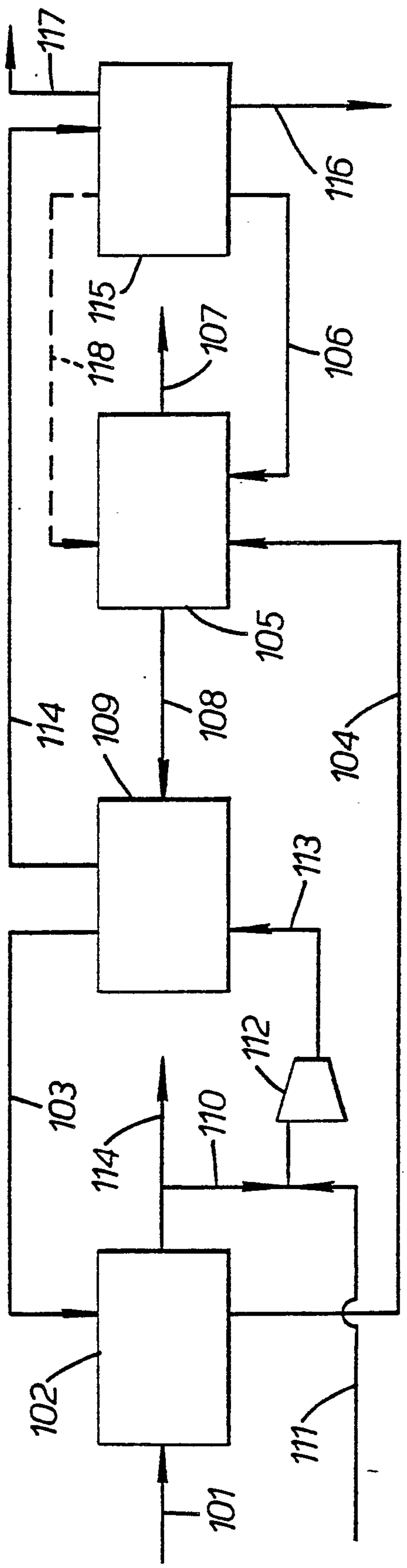

101
102
103
104
105
106
107
108
109
110
111
112
113
114
114
115
116
117
118


FIG. 2.

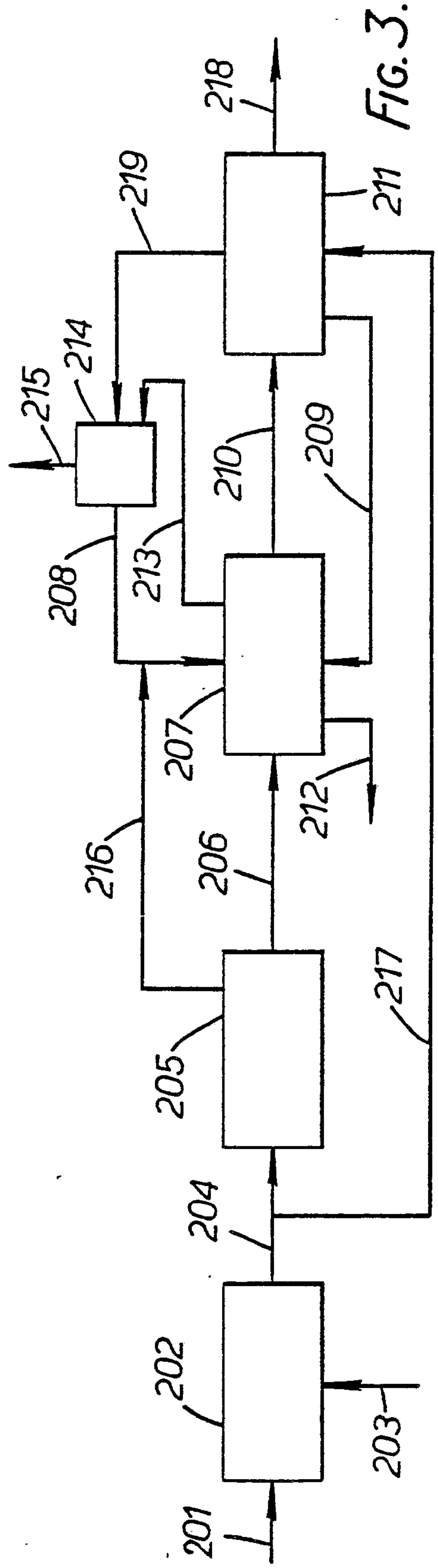

201
202
203
204
205
206
207
208
209
210
211
212
213
214
215
216
217
218
219


FIG. 3.